# EUROPEAN PATENT SPECIFICATION

(11) **EP 2 676 950 B1**
(45) Date of publication and mention of the grant of the patent: **18.04.2018**
(21) Application number: 12746983.1
(22) Date of filing: 02.02.2012
(51) Int. Cl.: C07C 263/18, C07C 265/04

(54) **STABILIZED ISOCYANATE GROUP-CONTAINING ETHYLENE-BASED UNSATURATED COMPOUND**
STABILISIERTE ISOCYANATGRUPPEN ENTHALTENDE UNGESÄTTIGTE VERBINDUNG AUF ETHYLENBASIS
COMPOSÉ ÉTHYLÉNIQUEMENT INSATURÉ COMPRENANT UN GROUPE ISOCYANATE STABILISÉ

(30) Priority: 15.02.2011 JP 2011029752
(43) Date of publication of application: 25.12.2013
(73) Proprietor: SHOWA DENKO K.K., Tokyo 105-8518 (JP)
(72) Inventor: NISHIMURA, Norihito, Aizuwakamatsu-shi, Fukushima 969-3431 (JP); YOROZUYA, Shinichi, Aizuwakamatsu-shi, Fukushima 969-3431 (JP); OHNO, Katsutoshi, Aizuwakamatsu-shi, Fukushima 969-3431 (JP)
(74) Representative: Strehl Schübel-Hopf & Partner
(86) International application number: PCT/JP2012/052383
(87) International publication number: WO 2012/111446

(56) References cited:
- EP-A1- 1 745 013
- EP-A2- 0 936 214
- WO-A1-2010/016540
- JP-A- 11 228 523
- JP-A- 2000 086 612
- JP-A- 2008 137 948
- JP-A- 2009 051 785
- JP-A- 2009 051 785

## Description

### TECHNICAL FIELD

The present invention relates to a stabilized isocyanate group-containing ethylenically unsaturated compound and more specifically relates to a stabilizing composition containing the compound and to a method for producing the compound.

### BACKGROUND ART

Since an ethylenically unsaturated compound is prone to be extremely polymerized by heat, light and other factors, a solid is often generated by polymerization in the steps of producing and purifying an ethylenically unsaturated compound and causes the failure of production facilities. When the solid adheres to a production tank, a distillation column, a pipe and the like in the steps of producing and purifying an ethylenically unsaturated compound, not only, e.g., clogging due to the solid and anchoring of mobile facilities occur but also the solid interferes with the production and purification of the ethylenically unsaturated compound. In addition, the removal of the solid adhering to related facilities is generally performed by operation by human power and therefore has poor operation efficiency, a long-term shutdown is thus unavoidable, and an economic loss is large. Also, the deterioration of product quality is also caused.

Although it is known that the addition of various solid polymerization inhibitors is effective as a method for preventing the polymerization of an ethylenically unsaturated compound, only the addition is insufficient and many polymerization preventing methods other than the method have been proposed or performed.

For example, there has been proposed a method of adding a compound, in which Michael addition of alcohol or (meth)acrylic acid to a (meth) acrylic acid ester occurs, in a low-boiling point impurity distilling step of a crude (meth)acrylic acid ester (Patent Literature 1). However, this method is less effective since alcohol in a Michael addition moiety is desorbed by heating to reproduce a (meth) acrylic group in the case of using the Michael adduct of an alcohol or is ineffective from the viewpoint of the prevention of polymerization since there is still a (meth) acrylic group which is polymerizable in the case of using the compound in which the Michael addition of (meth)acrylic acid occurs. Furthermore, the addition of various impurities having such different strictures is not preferred since a functionally adverse effect is caused when using the product to make a secondary product (such as a resin molding, a coating or an optical material).

In view of the above, the advent of a method for more effectively preventing the polymerization of an ethylenically unsaturated compound has been earnestly desired.

Further, there is a problem that the multimerization reaction (isocyanuration, allophanation or biuret reaction, etc.) of an isocyanate group or a discoloration reaction occurs in the case of the ethylenically unsaturated compound having an isocyanate group in the molecule. For example, the multimerization reaction may proceed during storing the product (ethylenically unsaturated compound having an isocyanate group), and, in this case, cloudiness, discoloration or decrease in purity is revealed just before using the product, so that a step is delayed due to, e.g., a wait for the delivery of an alternative. Any compounds effective for preventing these side reactions have not been currently present and the advent of the technology of preventing these side reactions has been earnestly desired.

### CITATION LIST

### PATENT LITERATURE

Patent Literature 1: JP-H9(1997)-301921 A

JP 2009 051785 A discloses a composition comprising an isocyanoacetic acid alkyl ester and a stabilizer selected from silver nitrate, an organic acid, an acid anhydride thereof, and the organic acid's ammonium salt.

### SUMMARY OF INVENTION

### TECHNICAL PROBLEM

In view of the conventional problems, an object of the present invention is to improve the stability of an ethylenically unsaturated compound having an isocyanate group in the molecule by preventing a polymerization, a multimerization reaction and a discoloration reaction of the ethylenically unsaturated compound.

### SOLUTION TO PROBLEM

As a result of performing extensive research in order to solve the problems, the present inventors found that an isocyanate group-containing ethylenically unsaturated compound is stabilized by a method of incorporating a certain stabilizing agent into the isocyanate group-containing ethylenically unsaturated compound, and the present invention was thus accomplished. The present invention includes, e.g., the following sections.
[1] A stabilizing composition for an isocyanate group-containing ethylenically unsaturated compound, comprising:
   an isocyanate group-containing ethylenically unsaturated compound (A) which comprises one or more isocyanate groups and one or more ethylenically unsaturated groups in the molecule; and a stabilizing agent (B) which is a compound in which at least one of the ethylenically unsaturated groups in the compound (A) is replaced with an alkyl halide group or an amino alkyl group which may have a substituent,
   wherein the compound (A) is 2-methacryloyloxyethyl isocyanate and the stabilizing agent (B) is 3'-chloro-2'-methylpropionyl-2-oxyethyl isocyanate.
[2] A method for producing a stabilized isocyanate group-containing ethylenically unsaturated compound, wherein, in a process for producing an isocyanate group-containing ethylenically unsaturated compound (A) which comprises one or more isocyanate groups and one or more ethylenically unsaturated groups in the molecule,
   a stabilizing agent (B) is incorporated into a raw material for producing the compound (A); and
   the stabilizing agent (B) is a compound in which at least one of the ethylenically unsaturated groups in the compound (A) is replaced with an alkyl halide group or an amino alkyl group which may have a substituent,
   wherein the compound (A) is 2-methacryloyloxyethyl isocyanate and the stabilizing agent (B) is 3'-chloro-2'-methylpropionyl-2-oxyethyl isocyanate.
[3] A method for producing a stabilized isocyanate group-containing ethylenically unsaturated compound, wherein, in a process for producing an isocyanate group-containing ethylenically unsaturated compound (A) which comprises one or more isocyanate groups and one or more ethylenically unsaturated groups in the molecule,
   a stabilizing agent (B) is added to the compound (A); and the stabilizing agent (B) is a compound in which at least one of the ethylenically unsaturated groups in the compound (A) is replaced with an alkyl halide group or an amino alkyl group which may have a substituent,
   wherein the compound (A) is 2-acryloyloxyethyl isocyanate and the stabilizing agent (B) is 3'-chloropropionyl-2-oxyethyl isocyanate, or
   wherein the compound (A) is 2-methacryloyloxyethyl isocyanate and the stabilizing agent (B) is 3'-chloro-2'-methylpropionyl-2-oxyethyl isocyanate.

### EFFECT OF THE INVENTION

In accordance with the present invention, an isocyanate group-containing ethylenically unsaturated compound can be effectively stabilized. Thus, there can be effectively prevented the generation of impurities in production, product storage or the like; the generation of a solid, the occurrence of cloudiness, discoloration and reduction in purity due to polymerization and multimerization; and the like.

### EMBODIMENT FOR CARRYING OUT THE INVENTION

The present invention relates to a stabilizing composition comprising a stabilized isocyanate group-containing ethylenically unsaturated compound and to a method for producing the compound. The present invention will be described in detail below.

As used herein, "ethylenically unsaturated compound" means a compound having at least one ethylenically unsaturated group in the molecule, and the ethylenically unsaturated group may have a substituent. "(Meth)acryloyl" means acryloyl or methacryloyl and also encompasses groups of which some of the hydrogen atoms are substituted. Further, hydrolyzable chlorine is an organic or inorganic chlorine compound that is generated during producing isocyanate and reacts with methanol to generate hydrogen chloride under the test conditions according to Japanese Industrial Standard JIS K1603-3: 2007 and is contained in a minute amount in an isocyanate group-containing ethylenically unsaturated compound.

### [Stabilizing Composition]

The stabilizing composition according to the present invention comprises an isocyanate group-containing ethylenically unsaturated compound (A) and a stabilizing agent (B).

### <Isocyanate Group-Containing Ethylenically Unsaturated Compound (A)>

The isocyanate group-containing ethylenically unsaturated compound (A) is 2-methacryloyloxyethyl isocyanate.

### <Stabilizing Agent (B)>

The stabilizing agent (B) according to the present invention is 3'-chloro-2'-methylpropioyl-2-oxyethyl isocyanate.

In one inventive method, compound (A) and stabilizing agent (B) are as defined in claim 3.

### <Contents of Stabilizing Agents (B)>

The content of the stabilizing agent (B) in the stabilizing composition of the present invention is preferably 0.005% by mass or more and 5% by mass or less, more preferably 0.1% by mass or more and 3% by mass or less. The content of less than 0.005% by mass may cause a sufficient stabilization effect to be prevented from being expressed while the case of the content of more than 5% by mass is not preferred in terms of economical efficiency.

### <Another Component>

As used herein, "polymerization" encompasses the generation of a solid (in popcorn, glass or gel form, etc.), such as the generation of, e.g., a granular or massive solid as a polymer or the solidification of a liquid phase portion as well as the state in which a compound of which the multimerization or oligomerization occurs due to the polymerization reaction of the compound (A) is not generated as a solid but is dissolved in the liquid of the compound (A). Such a case can be confirmed by quantitating the decrease amount of the compound (A) or the production amount of an oligomer by a common analytical method (e.g., gas chromatography, high performance liquid chromatography or the like).

### <Polymerization>

As used herein, "polymerization" encompasses the generation of a solid (in popcorn, glass, or gel form, etc.), such as the generation of, e.g., a granular or massive solid as a polymer or the solidification of a liquid phase portion as well as the state in which a compound of which the multimerization or oligomerization occurs due to the polymerization reaction of the compound (A) is not generated as a solid but is dissolved in the liquid of the compound (A). Such a case can be confirmed by quantitating the decrease amount of the compound (A) or the production amount of an oligomer by a common analytical method (e.g., gas chromatography, high performance liquid chromatography, or the like).

### [Method for Producing Stabilized Isocyanate Group-Containing Ethylenically Unsaturated Compound]

The methods for obtaining a stabilizing composition comprising the compound (A) and the stabilizing agent (B) (method for producing a stabilized isocyanate group-containing ethylenically unsaturated compound) are:
i) a method of adding a separately synthesized stabilizing agent (B) to a compound (A) or recycling a stabilizing agent (B) collected in a process for producing a compound (A) to add it to a compound (A);
ii) a method of incorporating a stabilizing agent (B) into a raw material for producing a compound (A) or a method of incorporating a precursor for a stabilizing agent (B) into a raw material for producing a compound (A) and making a reaction proceed with the raw material to generate a stabilizing agent (B), in a process for producing a compound (A).

The precursor is not particularly limited as long as the precursor is a compound that is finally converted into a stabilizing agent (B) when being subjected to a reaction by being incorporated into a raw material for a compound (A) in a process for producing a compound (A).

Specific examples of i) as described above include a method of adding separately synthesized and purified 3'-chloropropionyl-2-oxyethyl isocyanate to 2-acryloyloxyethyl isocyanate before or after distillation.

Specific examples of ii) as described above include:
ii-1) a method of not completely terminating a reaction but adjusting the reaction so that a certain amount of 3'-chloropropionyl-2-oxyethyl isocyanate remains in a product when 2-acryloyloxyalkyl isocyanate is produced from 2-chloropropionyloxyethyl isocyanate through a dehydrochlorination step;
ii-2) a method of incorporating 3'-chloro-2'-methylpropionyl-2-oxyethyl isocyanate functioning as a stabilizing agent into a final product by incorporating a specific amount of 3-chloro-2-methylpropionic acid into methacrylic acid as a raw material to perform the reaction thereof when methacrylic acid is reacted with phosgene to generate methacrylic acid chloride, which is reacted with ethanolamine hydrochloride to obtain methacryloyloxyethylamine hydrochloride, followed by producing 2-methacryloyloxyethyl isocyanate by the step of isocyanation of amine; and the like.

When the stabilizing agent (B') is further added, the stabilizing agent (B') can be incorporated by the same method as in the case of the stabilizing agent (B) described above.

### <Mechanism of Stabilization>

The detailed mechanism of the stabilization of the compound (A) according to the present invention is unclear at present. As the presumption at present, there is considered the effect that the stabilizing agent (B) in itself or hydrogen halide or amine partly dissociated from the stabilizing agent (B) scavenges a basic substance or radical to contribute to the stabilization. Particularly, it is considered that there is the effect of contributing to the stabilization of an isocyanate group in the compound (A) to suppress a multimerization reaction or a discoloration reaction. Furthermore, when the stabilizing agent (B') is contained, an overall double bond equivalent is decreased and the stabilizing agent (B') receives a generated heat radical to be able to decrease the risk of the destabilization due to polymerization of the unsaturated ethylenic double bond of the compound (A). Further, examples of the points include such an advantage that the physical properties of a coating film or the like during use thereof as a resin composition or after curing are not adversely affected since the structures of the compound (A), the stabilizing agent (B) and the stabilizing agent (B') are similar.

### EXAMPLES

The present invention will be described below based on examples.

### [Stabilization of Isocyanate Group-Containing Ethylenically Unsaturated Compound on Presumption of Heating Reaction Conditions]

### [Example 1]

In a glass flask with a capacity of 300 mL, including a cooling condenser (which had an outlet opened at atmospheric pressure and was equipped with a granular calcium chloride pipe) and a thermometer, 100 g of 2-acryloyloxyethyl isocyanate ("KarenzAOI" (registered trademark) manufactured by Showa Denko K.K., purity of 99.7% by gas chromatography, hereinafter also referred to as "AOI monomer") as the isocyanate group-containing ethylenically unsaturated compound (A) and 1 g of 3'-chloropropionyl-2-oxyethyl isocyanate as the stabilizing agent (B) for the compounds (A) were put and heated in an oil bath at 100 °C for 8 hours. When the amount of a remaining AOI monomer was quantitated by gas chromatography after finishing the heating, the amount was decreased by about 0.2% based on the loading amount and the isocyanurate body of the AOI monomer was not generated. In this case, the appearance of the internal liquid in the flask was transparent without change. The analytical conditions by the gas chromatography are as follows:

### <Analytical Conditions>

Column: J & W Co., DB-1 (length of 30 m, inner diameter of 0.32 mm, film thickness of 1 µm)
Sample injector temperature: 300°C
Detector temperature: 300°C
Detector: FID (hydrogen flame type detector)
Temperature-raising program: 50°C → 10°C/min → 320°C (5 min hold)
Flow rate: 1.2 mL/min
Sample dilution solvent: methylene chloride

### [Comparative Example 1]

Heating was performed by the same method as in Example 1 except that 3'-chloropropionyl-2-oxyethyl isocyanate was not used. After finishing the heating, the amount of a remaining AOI monomer was evaluated by gas chromatograph. As a result, the amount was decreased by about 8.1% based on the amount of a loaded AOI monomer and the generation of a type of an isocyanurate body, in which isocyanate groups of three molecules of the AOI monomer were reacted, was observed. In this case, the internal liquid in the flask became cloudy.

### [Reference Example 1]

The amount of a remaining AOI monomer was evaluated by the same method as in Example 1 except that 1 g of 2-n-propionyloxyethyl isocyanate was used instead of 1 g of 3'-chloropropionyl-2-oxyethyl isocyanate as the stabilizing agent (B). As a result, the amount was decreased by 0.3% based on the loading amount. In this case, no change of the appearance was seen.

### [Comparative Reference Example 1]

The amount of a remaining AOI monomer was evaluated by the same method as in Reference Example 1 except that 2-n-propionyloxyethyl isocyanate was not used. As a result, the amount was decreased by about 2.3% based on the loading amount. In this case, white fine particles were generated in the internal liquid in the flask.

### [Reference Example 2]

The amount of a remaining MOI monomer was evaluated by the same method as in Reference Example 1 except that 100 g of 2-methacryloyloxyethyl isocyanate ("KarenzMOI" (registered trademark) manufactured by Showa Denko K.K., purity of 99.7% by gas chromatography, hereinafter also referred to as "MOI monomer") was used instead of 100 g of the AOI monomer as the isocyanate group-containing ethylenically unsaturated compound (A). As a result, the amount was decreased by about 0.6% based on the loading amount. In this case, no change of the appearance was seen.

### [Comparative Reference Example 2]

The amount of a remaining MOI monomer was evaluated by the same method as in Reference Example 2 except that 2-n-propionyloxyethyl isocyanate was not used. As a result, the amount was decreased by about 3.2% based on the loading amount. In this case, white fine particles were generated in the internal liquid in the flask.

### [Reference Example 3]

### (3-1) Synthesis of 2-acryloyloxyethyl isocyanate reaction liquid

### (Step 1)

In a four-neck flask equipped with a thermometer, a cooling pipe, a gas supply pipe and a stirring apparatus, 200 g of acrylic acid and 4 g of dimethylformamide were put and heated to 70°C, and 400 g of phosgene was supplied for 40 hours. After the supply, excess phosgene was removed, followed by distilling off the resultant under reduced pressure (60°C/3 kPa). There was separated 20 g of the first fraction, and 200 g of 3-chloropropionic acid chloride was obtained as a main fraction.

### (Step 2)

In a four-neck flask equipped with a thermometer, a cooling pipe, a gas supply pipe and a stirring apparatus, 750 mL of toluene and 75 g (1.23 mol) of ethanolamine were put and heated to 90°C, and about 60 g of hydrogen chloride gas was supplied. Then, 177 g (1.39 mol) of 3-chloropropionic acid chloride obtained in the step 1 was dropwise added for 270 minutes and heated at 90°C for 1 hour. Thereafter, 240 g (2.43 mol) of phosgene was supplied for 12 hours. Then, dissolved phosgene and toluene were removed to obtain 241 g (content of 81.0% by mass by gas chromatography, 1.10 mol as purity) of a 3-chloropropionic acid (2-isocyanatoethyl) ester reaction liquid.

### (Step 3)

In a three-necked flask, 670 mL of toluene, 241 g (1.10 mol) of the 3-chloropropionic acid (2-isocyanatoethyl) ester reaction liquid obtained in the step 2 and 134 g (1.31 mol) of triethylamine (boiling point; 89.4°C) were put, and heated and stirred at 50°C for 6 hours. After stirring, the resultant was cooled to room temperature, and generated triethylamine hydrochloride was filtrated. Then, excess triethylamine and toluene were distilled off to obtain 183 g of a reaction liquid of 2-acryloyloxyethyl isocyanate (concentration of 78.8% by mass by gas chromatography, 1.02 mol as purity).

### (3-2) Stabilization of 2-acryloyloxyethyl isocyanate reaction liquid

The amount of a remaining AOI monomer was evaluated by the same method as in Example 1 except that 127 g of the reaction liquid (78.8% as purity) of 2-acryloyloxyethyl isocyanate (78.8% as purity, the content of 2-acryloyloxyethyl isocyanate by gas chromatograph was 100 g) was used instead of 100 g of "KarenzAOI" as the isocyanate group-containing ethylenically unsaturated compound (A) and 2-n-propionyloxyethyl isocyanate was used instead of 1 g of 3'-chloropropionyl-2-oxyethyl isocyanate as the stabilizing agent (B). As a result, the amount was decreased by about 0.4% based on the loading amount. In this case, the appearance of the internal liquid in the flask was transparent without change.

### [Comparative Reference Example 3]

The amount of a remaining AOI monomer was evaluated by the same method as in Reference Example 3 except that 2-n-propionyloxyethyl isocyanate was not used. As a result, the amount was decreased by about 7.3% based on the loading amount. In this case, white fine particles were generated and floated in the internal liquid in the flask.

### [Example 2]

### (2-1) Synthesis of 2-acryloyloxyethyl isocyanate reaction liquid containing 3'-chloropropionyl-2-oxyethyl isocyanate

By the same method as in Reference Example 3 except that the amount of triethylamine used in the step 3 of Reference Example 3 was reduced to 111 g (1.10 mol) and 3'-chloropropionyl-2-oxyethyl isocyanate was reacted so as to partly remain, 183 g of a reaction liquid of 2-acryloyloxyethyl isocyanate was obtained. In analysis by gas chromatography, 2-acryloyloxyethyl isocyanate in a content of 78.0% by mass and 1.01 mol as purity and 3-chloropropionic acid (2-isocyanatoethyl) ester in 0.7% by mass and 0.01 mol as purity were contained.

### (2-2) Evaluation of stability of 2-acryloyloxyethyl isocyanate reaction liquid

Since the 2-acryloyloxyethyl isocyanate reaction liquid synthesized in (2-1) as described above had already contained 3'-chloropropionyl-2-oxyethyl isocyanate as the stabilizing agent (B), the amount of a remaining AOI monomer was evaluated by the method described in Example 1 using the reaction liquid. As a result, the amount of AOI was decreased by about 0.6% based on the loading amount. In this case, no change of the appearance was seen.

### [Reference Example 4]

### (4-1) Synthesis of 2-acryloyloxyethyl isocyanate reaction liquid containing 2-n-propionyloxyethyl isocyanate

By the same method as in Reference Example 3 except that a mixture of 1 g of propionic acid with 199 g of acrylic acid was used instead of 200 g of acrylic acid in the step 1 of Reference Example 3, 183 g of a reaction liquid of 2-acryloyloxyethyl isocyanate was obtained. In analysis by gas chromatography, 2-acryloyloxyethyl isocyanate in a content of 77.9% by mass and 1.01 mol as purity and 2-n-propionyloxyethyl isocyanate in 0.7% by mass and 0.01 mol as purity were contained.

### (4-2) Evaluation of stability of 2-acryloyloxyethyl isocyanate reaction liquid

Since the 2-acryloyloxyethyl isocyanate reaction liquid synthesized in (4-1) as described above had already contained 2-n-propionyloxyethyl isocyanate as the stabilizing agent (B), the amount of a remaining AOI monomer was evaluated by the method described in Example 1 using the reaction liquid. As a result, the amount of AOI was decreased by about 0.7% based on the loading amount. In this case, no change of the appearance was seen.

### [Example 3]

The amount of a remaining MOI monomer was evaluated by the same method as in Example 1 except that 100 g of 2-methacryloyloxyethyl isocyanate ("KarenzMOI" (registered trademark) manufactured by Showa Denko K.K., purity of 99.7% by gas chromatography) was used instead of 100 g of the AOI monomer as the isocyanate group-containing ethylenically unsaturated compound (A) and 1 g of 3'-chloro-2'-methylpropionyl-2-oxyethyl isocyanate was used instead of 1 g of 3'-chloropropionyl-2-oxyethyl isocyanate as the stabilizing agent (B). As a result, the amount was decreased by about 0.4% based on the loading amount. In this case, the appearance of the internal liquid in the flask was transparent without change.

### [Comparative Example 2]

The amount of a remaining MOI monomer was evaluated by the same method as in Example 3 except that 3'-chloro-2'-methylpropionyl-2-oxyethyl isocyanate was not used. As a result, the amount was decreased by about 6.9% based on the loading amount and the generation of the isocyanurate body of MOI was observed. In this case, the internal liquid in the flask became cloudy.

### [Stabilization of Isocyanate Group-Containing Ethylenically Unsaturated Compound after Distillation and Storage]

### [Example 4]

A reaction liquid of 2-acryloyloxyethyl isocyanate was prepared using the synthesis method of Reference Example 3. Using 128 g of the reaction liquid (the content of 2-acryloyloxyethyl isocyanate by gas chromatograph was 100 g), 1 g of 3'-chloropropionyl-2-oxyethyl isocyanate and 1 g of phenothiazine as a polymerization inhibitor were added thereto, followed by carrying out simple distillation to obtain 68 g of a colorless and transparent fraction. The concentration of 2-acryloyloxyethyl isocyanate in the fraction was 99.7% by mass. Further, the quantitative value of 3'-chloropropionyl-2-oxyethyl isocyanate in the fraction was 0.2 g. This fraction was filled into a glass bottle, was stored in a constant-temperature bath, kept at about 60°C, for 7 days, and was thereafter analyzed. As a result, the concentration of 2-acryloyloxyethyl isocyanate was 99.7% by mass without change and the appearance thereof also remained colorless and transparent. In this case, the generation of impurities was not confirmed in analysis by gas chromatograph.

### [Comparative Example 3]

In the same manner as in Example 4 except that 1 g of 3'-chloropropionyl-2-oxyethyl isocyanate was not used, simple distillation was carried out to obtain 68 g of a colorless and transparent fraction. The concentration of 2-acryloyloxyethyl isocyanate in the fraction was 99.5% by mass. This fraction was filled in a glass bottle, was stored in a constant-temperature bath, kept at about 60°C, for 7 days in the same manner as in Example 4, and was thereafter analyzed. As a result, the concentration of 2-acryloyloxyethyl isocyanate was decreased to 98.2% by mass and the appearance thereof became cloudy liquid. In this case, the generation of impurities appearing to be isocyanurate and biuret bodies and the like was confirmed in analysis by gas chromatograph.

### [Example 5]

In the same manner as in Example 4 except that 1 g of 3'-chloropropionyl-2-oxyethyl isocyanate was not used, simple distillation was carried out to obtain 68 g of a colorless and transparent fraction. The concentration of 2-acryloyloxyethyl isocyanate in the fraction was 99.5% by mass. When 0.2 g of 3'-chloropropionyl-2-oxyethyl isocyanate was added to this fraction to analyze the resultant by gas chromatograph, the concentration of 2-acryloyloxyethyl isocyanate was 99.2% by mass and was decreased by the theoretical amount of dilution with 3'-chloropropionyl-2-oxyethyl isocyanate. This fraction was filled in a glass bottle, was stored in a constant-temperature bath, kept at about 60°C, for 7 days in the same manner as in Example 4, and was thereafter analyzed. As a result, the concentration of 2-acryloyloxyethyl isocyanate was 99.2% by mass without change and the appearance thereof remained colorless and transparent. In this case, the generation of impurities was not confirmed in the analysis by the gas chromatograph.

## Claims

1. A stabilizing composition for an isocyanate group-containing ethylenically unsaturated compound, comprising:
an isocyanate group-containing ethylenically unsaturated compound (A) which comprises one or more isocyanate groups and one or more ethylenically unsaturated groups in the molecule; and
a stabilizing agent (B) which is a compound in which at least one of the ethylenically unsaturated groups in the compound (A) is replaced with an alkyl halide group or an amino alkyl group which may have a substituent,
wherein the compound (A) is 2-methacryloyloxyethyl isocyanate and the stabilizing agent (B) is 3'-chloro-2'-methylpropionyl-2-oxyethyl isocyanate.

2. A method for producing a stabilized isocyanate group-containing ethylenically unsaturated compound, wherein,
in a process for producing an isocyanate group-containing ethylenically unsaturated compound (A) which comprises one or more isocyanate groups and one or more ethylenically unsaturated groups in the molecule,
a stabilizing agent (B) is incorporated into a raw material for producing the compound (A); and
the stabilizing agent (B) is a compound in which at least one of the ethylenically unsaturated groups in the compound (A) is replaced with an alkyl halide group or an amino alkyl group which may have a substituent,
wherein the compound (A) is 2-methacryloyloxyethyl isocyanate and the stabilizing agent (B) is 3'-chloro-2'-methylpropionyl-2-oxyethyl isocyanate.

3. A method for producing a stabilized isocyanate group-containing ethylenically unsaturated compound, wherein,
in a process for producing an isocyanate group-containing ethylenically unsaturated compound (A) which comprises one or more isocyanate groups and one or more ethylenically unsaturated groups in the molecule,
a stabilizing agent (B) is added to the compound (A); and
the stabilizing agent (B) is a compound in which at least one of the ethylenically unsaturated groups in the compound (A) is replaced with an alkyl halide group or an amino alkyl group which may have a substituent,
wherein the compound (A) is 2-acryloyloxyethyl isocyanate and the stabilizing agent (B) is 3'-chloropropionyl-2-oxyethyl isocyanate, or
wherein the compound (A) is 2-methacryloyloxyethyl isocyanate and the stabilizing agent (B) is 3'-chloro-2'-methylpropionyl-2-oxyethyl isocyanate.

## Patentansprüche

1. Stabilisierungszusammensetzung für eine isocyanatgruppenhaltige, ethylenisch ungesättigte Verbindung, welche umfasst: eine isocyanatgruppenhaltige, ethylenisch ungesättigte Verbindung (A), die eine oder mehrere Isocyanatgruppen und eine oder mehrere ethylenisch ungesättigte Gruppen im Molekül enthält; und
ein Stabilisierungsmittel (B), das eine Verbindung ist, in der mindestens eine der ethylenisch ungesättigten Gruppen in der Verbindung (A) durch eine Alkylhalogenidgruppe oder eine Aminoalkylgruppe, die einen Substituenten aufweisen kann, ersetzt ist,
wobei die Verbindung (A) 2-Methacryloyloxyethylisocyanat ist und das Stabilisierungsmittel (B) 3'-Chlor-2'-methylpropionyl-2-oxyethylisocyanat ist.

2. Verfahren zum Herstellen einer stabilisierten, isocyanatgruppenhaltigen ethylenisch ungesättigten Verbindung, wobei in einem Verfahren zum Herstellen einer isocyanatgruppenhaltigen, ethylenisch ungesättigten Verbindung (A), die eine oder mehrere Isocyanatgruppen und eine oder mehrere ethylenisch ungesättigte Gruppen im Molekül enthält,
ein Stabilisierungsmittel (B) in ein Ausgangsmaterial zum Herstellen der Verbindung (A) einverleibt wird; und
das Stabilisierungsmittel (B) eine Verbindung ist, in der mindestens eine der ethylenisch ungesättigten Gruppen in der Verbindung (A) durch eine Alkylhalogenidgruppe oder eine Aminoalkylgruppe, die einen Substituenten aufweisen kann, ersetzt ist,
wobei die Verbindung (A) 2-Methacryloyloxyethylisocyanat ist und das Stabilisierungsmittel (B) 3'-Chlor-2'-methylpropionyl-2-oxyethylisocyanat ist.

3. Verfahren zum Herstellen einer stabilisierten, isocyanatgruppenhaltigen, ethylenisch ungesättigten Verbindung, wobei in einem Verfahren zum Herstellen einer isocyanatgruppenhaltigen, ethylenisch ungesättigten Verbindung, die eine oder mehrere Isocyanatgruppen und eine oder mehrere ethylenisch ungesättigte Gruppen im Molekül enthält, ein Stabilisierungsmittel (B) zu der Verbindung (A) gegeben wird; und
das Stabilisierungsmittel (B) eine Verbindung ist, in der mindestens eine der ethylenisch ungesättigten Gruppen in der Verbindung (A) durch eine Alkylhalogenidgruppe oder eine Aminoalkylgruppe, die einen Substituenten aufweisen kann, ersetzt ist,
wobei die Verbindung (A) 2-Acryloyloxyethylisocyanat ist und das Stabilisierungsmittel (B) 3'-Chlorpropionyl-2-oxyethylisocyanat ist, oder
wobei die Verbindung (A) 2-Methacryloyloxyethylisocyanat ist, und das Stabilsierungsmittel (B) 3'-Chlor-2'-methylpropionyl-2-oxyethylisocyanat ist.

## Revendications

1. Composition stabilisée d'un composé éthyléniquement insaturé comprenant un groupe isocyanate, comprenant :
un composé éthyléniquement insaturé comprenant un groupe isocyanate (A) qui comprend un ou plusieurs groupes isocyanate et un ou plusieurs groupes éthyléniquement insaturés par molécule ; et
un agent stabilisant (B) qui est un composé dans lequel au moins l'un des groupes éthyléniquement insaturés du composé (A) est remplacé par un groupe halogénure d'alkyle ou un groupe aminoalkyle qui peut présenter un substituant,
dans laquelle le composé (A) est l'isocyanate de 2-méthacryloyloxyéthyle et l'agent stabilisant (B) est l'isocyanate de 3'-chloro-2'-méthylpropionyl-2-oxyéthyle.

2. Procédé de production d'un composé éthyléniquement insaturé comprenant un groupe isocyanate stabilisé, dans lequel, dans un procédé de production d'un composé éthyléniquement insaturé comprenant un groupe isocyanate (A) qui comprend un ou plusieurs groupes isocyanate et un ou plusieurs groupes éthyléniquement insaturés par molécule,
un agent stabilisant (B) est incorporé dans une matière première pour produire le composé (A) ; et
l'agent stabilisant (B) est un composé dans lequel au moins l'un des groupes éthyléniquement insaturés du composé (A) est remplacé par un groupe halogénure d'alkyle ou un groupe aminoalkyle qui peut présenter un substituant,
dans lequel le composé (A) est l'isocyanate de 2-méthacryloyloxyéthyle et l'agent stabilisant (B) est l'isocyanate de 3'-chloro-2'-méthylpropionyl-2-oxyéthyle.

3. Procédé de production d'un composé éthyléniquement insaturé comprenant un groupe isocyanate stabilisé, dans lequel, dans un procédé de production d'un composé éthyléniquement insaturé comprenant un groupe isocyanate (A) qui comprend un ou plusieurs groupes isocyanate et un ou plusieurs groupes éthyléniquement insaturés par molécule,
un agent stabilisant (B) est ajouté au composé (A) ; et
l'agent stabilisant (B) est un composé dans lequel au moins l'un des groupes éthyléniquement insaturés du composé (A) est remplacé par un groupe halogénure d'alkyle ou un groupe aminoalkyle qui peut présenter un substituant,
dans lequel le composé (A) est l'isocyanate de 2-acryloyloxyéthyle et l'agent stabilisant (B) est l'isocyanate de 3'-chloropropionyl-2-oxyéthyle, ou
dans lequel le composé (A) est l'isocyanate de 2-méthacryloyloxyéthyle et l'agent stabilisant (B) est l'isocyanate de 3'-chloro-2'-méthylpropionyl-2-oxyéthyle.
